# EUROPEAN PATENT APPLICATION

(11) **EP 1 563 864 A2**
(43) Date of publication of application: **17.08.2005**
(21) Application number: 05100647.6
(22) Date of filing: 24.01.1997
(51) Int. Cl.: A61M 15/00

(54) **Inhalation device**

(30) Priority: 25.01.1996 US 599508
(62) Divisional of application: 97904803.0
(71) Applicant: Microdose Technologies Inc., Monmouth Jct., NJ 08852 (US)
(72) Inventor: Abrams, Andrew L., 06880, Connecticut (US); Gumaste, Anand, 08691, New Jersey (US)
(74) Representative: Poulin, Gérard

(57) **Abstract**

An inhaler that utilizes vibration to facilitate suspension of powder into a gas is provided. The inhaler includes a piezoelectric vibrator for vibrating the powder. Deaggregated powder is suspended in a secondary air flow passageway before entering a primary air flow passageway.

A controller is provided for controlling supply of actuating electricity to the vibrator so as to cause the powder to vibrate in such a way to optimally suspend at least a portion of the powder into the gas. The controller may include a user-actuable control for permitting the user to select the vibration frequencies and/or amplitudes for optimally suspending in the gas the type of powder currently being used in the inhaler.

## Description

The present invention relates generally to the field of inhalation devices, and more specifically, to inhalation devices that utilize vibration to facilitate suspension of powder (e.g., powdered medication) into an inhaled gas stream (e.g., of inhaled air). Particular utility for the present invention is found in the area of facilitating inhalation of powdered medications (e.g., bacterial vaccines, sinusitis vaccines, antihistaminic agents, vaso-constricting agents, anti-bacterial agents, anti-asthmatic agents, theophylline, aminophylline, di-sodium cromolyn, etc.), although other utilities are contemplated, including other medicament applications.

Certain diseases of the respiratory tract are known to respond to treatment by the direct application of therapeutic agents. As these agents are most readily available in dry powdered form, their application is most conveniently accomplished by inhaling the powdered material through the nose or mouth. This powdered form results in the better utilization of the medicament in that the drug is deposited exactly at the site desired and where its action may be required; hence, very minute doses of the drug are often equally as efficacious as larger doses administered by other means, with a consequent marked reduction in the incidence of undesired side effects and medicament cost. Alternatively, the drug in this form may be used for treatment of diseases other than those of the respiratory system. When the drug is deposited on the very large surface areas of the lungs, it may be very rapidly absorbed into the blood stream; hence, this method of application may take the place of administration by injection, tablet, or other conventional means.

It is the opinion of the pharmaceutical industry that the bioavailability of the drug is optimum when the drug particles delivered to the respiratory tract are between 1 to 5 microns in size. When the drug particles need to be in this size range the dry powder delivery system needs to address a number of issues:
(1) Small size particles develop an electrostatic charge on themselves during manufacturing and storage. This causes the particles to agglomerate or aggregate, resulting in clusters of particles which have an effective size greater than 5 microns. The probability of these large clusters making it to the deep lungs then decreases. This would result in a lower percentage of the packaged drug being available to the patient for absorption.
(2) The amount of active drug that needs to be delivered to the patient may be of the order of 10s of micrograms. For example, albuterol, in the case of a drug used in asthma, this is usually 25 to 50 micrograms. Current manufacturing equipment can effectively fill milligrams of drug with acceptable accuracy. So the standard practice is to mix the active drug with a filler or bulking agent such as lactose. This additive also makes the drug "easy to flow". This filler is also called a carrier since the drug particles also stick to these particles through electrostatic or chemical bonds. These carrier particles are very much larger than the drug particles in size. The ability of the dry powder inhaler to separate drug from the carrier is an important performance parameter in the effectiveness of the design.
(3) Active drug particles with sizes greater than 5 microns will be deposited either in the mouth or throat. This introduces another level of uncertainty since the bioavailability and absorption of the drug in these locations is different from the lungs. Dry powder inhalers need to minimize the drug deposited in these locations to reduce the uncertainty associated with the bio-availability of the drug.

Prior art dry powder inhalers (DPIs) usually have a means for introducing the drug (active drug plus carrier) into a high velocity air stream. The high velocity air-stream is used as the primary mechanism for breaking up the cluster of micronized particles or separating the drug particles from the carrier. Several inhalation devices useful for dispensing this powder form of medicament are known in the prior art. For example, in U.S. Patent Nos. 3,507,277; 3,518,992; 3,635,219; 3,795,244; and 3,807,400, inhalation devices are disclosed having means for piercing of a capsule containing a powdered medicament, which upon inhalation is drawn out of the pierced capsule and into the user's mouth. Several of these patents disclose propeller means, which upon inhalation aid in dispensing the powder out of the capsule, so that it is not necessary to rely solely on the inhaled air to suction powder from the capsule. For example, in U.S. Patent No. 2,517,482, a device is disclosed having a powder containing capsule placed in a lower chamber before inhalation, where it is pierced by manual depression of a piercing pin by the user. After piercing, inhalation is begun and the capsule is drawn into an upper chamber of the device where it moves about in all directions to cause a dispensing of powder through the pierced holes and into the inhaled air stream. U.S. Patent No. 3,831,606 discloses an inhalation device having multiple piercing pins, propeller means, and a self-contained power source for operating the propeller means via external manual manipulation, so that upon inhalation the propeller means aids in dispensing the powder into the stream of inhaled air. See also U.S. Patent No. 5,458,135.

These prior art devices present several problems and possess several disadvantages which are remedied by the inhalation devices of the present invention. For instance, these prior art devices require that the user exert considerable effort in inhalation to effect dispensing or withdrawal of powder from a pierced capsule into the inhaled air stream. With these prior art devices, suction of powder through the pierced holes in the capsule caused by inhalation generally does not withdraw all or even most of the powder out of the capsule, thus causing a waste of the medicament. Also, such prior art devices result in uncontrolled amounts or clumps of powdered material being inhaled into the user's mouth, rather than a constant inhalation of controlled amounts of finely dispersed powder.

The above description of the prior art is taken largely from U.S. Pat. No. 3,948,264 to Wilke et al, who disclose a device for facilitating inhalation of a powdered medication that includes a body portion having primary and secondary air inlet channels and an outlet channel. The secondary inlet channel provides an enclosure for a capsule containing the powdered medication and the outlet channel is formed as a mouthpiece protruding from the body. A capsule piercing structure is provided, which upon rotation puts one or more holes in the capsule so that upon vibration of the capsule by an electro-mechanical vibrator, the powdered drug may be released from the capsule. The piercing means disclosed in Wilke et al includes three radially mounted, spring-biased piercing needles mounted in a trochoidal chamber. Upon hand rotation of the chamber, simultaneous inward radial motion of the needles pierces the capsule. Further rotation of the chamber allows the needles to be retracted by their spring mountings to their original positions to withdraw the needles from the capsule. The electromechanical vibrator includes, at its innermost end, a vibrating plunger rod which projects into the intersection of the inlet channel and the outlet channel. Connected to the plunger rod is a mechanical solenoid buzzer for energizing the rod to vibrate. The buzzer is powered by a high energy electric cell and is activated by an external button switch. According to Wilke et al, upon inhalation through outlet channel 3 and concurrent pressing of switch 10d to activate the electromechanical vibrating means 10, air is sucked through inlet channels 4 and 12 and the air stream through the secondary inlet channel 4 raises the capsule up against the vibrating plunger rod 10a. The capsule is thus vibrated rapidly with powder being fluidized and dispensed from the pierced holes therein. (This technique is commonly used in manufacturing for dispensing powder through a hopper where the hopper is vibrated to fluidize the powder and move it through the hopper outlet. The pierced holes in the capsule represent the hopper outlet.) The air stream through inlet channel 4 and 12 aids in withdrawal of powder from the capsule and carries this powder through the outlet channel 3 to the mouth of the user." (Wilke et al, column 3, lines 45-55). Wilke et al further discloses that the electromechanical vibrator means may be placed at a right angle to the inlet chamber and that the amplitude and frequency of vibration may be altered to regulate dispensing characteristics of the inhaler.

Thus, as noted above, the vibrator in Wilke et al's disclosed inhaler is an electromechanical device consisting of a rod driven by a solenoid buzzer. (This electromechanical means may be a motor driving a cam [Col. 4, Line 40]). A disadvantage of the inhaler implementation as disclosed by Wilke is the relatively large mechanical movement required of the rod to effectively vibrate the capsule. The large movement of the rod, usually around 100s of microns, is necessary due to the elasticity of the capsule walls and inertia of the drug and capsule.

Moreover, solenoid buzzers typically have operating frequencies less than 5 Khz. This operating frequency tends to be noisy and therefore is not desirable when incorporated into a dry powder inhaler from a patient's perspective. A further disadvantage of the electrochemical actuators of Wilke is the requirement for a high energy source (Wilke et al, Col. 3, line 38), thus requiring a large battery source or frequent changes of the battery pack for portable units. Both these features are not desirable from a patient safety and "ease of use" standpoint.

The inhaler of Wilke et al is primarily intended to reduce the amount of powder left behind in the capsule relative to other inhalers cited in the patent disclosure. (Wilke et al, Col. 4, lines 59-68, Col. 5, lines 1-48). However, Wilke et al does not address the need to deaggragate the powder into particle sizes or groups less than 6 microns in size as is required for effective delivery of the medication to the lungs; rather Wilke et al, like the prior art inhalers continues to rely on the air stream velocity to deaggregate the powder ejected into the air stream, into particle sizes suitable for delivery to the lungs.

Another prior art inhalation device is disclosed in Bums et al U.S. Patent No. 5,284,133. In this device, a liquid medication is atomized by an ultrasonic device such as a piezo element (Bums et al, Col. 10, lines 36-51). A stream of air, usually at a high velocity, or a propellant then carries the atomized particles, to the patient. The energy required to atomize the liquid medication in the nebulizer is prohibitively high, making this approach for the delivery of drugs to the lungs only feasible as a desk top unit. The high voltage requirements to drive the piezo, to produce the necessary mechanical displacements, also severely effects the weight and size of the device. It is also not obvious that the nebulizer operating principles can be applied to the dry powder inhalers for delivery or powder medication to the lungs.

The prior art devices therefore have a number of disadvantages which makes them less than desirable for the delivery of dry powder to the lungs. Some of these disadvantages are:
- The performance of the prior art inhalers depends on the flowrate generated by the user. Lower flowrate does not result in the powder being totally deaggregated and hence adversely affects the dose delivered to the patient.
- Inconsistency in the bioavailability of the drugs from dose-to-dose because of lack of consistency in the deaggregation process.
- Large energy requirements for driving the electromechanical based inhalers which increases the size of the devices making them unsuitable for portable use.

Thus, it is the general object of the present invention to provide an inhaler that utilizes vibration to facilitate suspension of powder into a gas that overcomes the aforesaid and other disadvantages and drawbacks of the prior art. Accordingly, one embodiment of the inhaler of the present invention includes a piezoelectric vibrator for vibrating the powder. A controller is provided for controlling supply (i.e., amplitude and/or frequency) of actuating electricity to the vibrator so as to cause vibration of the powder that is adapted to optimally suspend at least a portion of the powder into the gas. In this embodiment of the present invention, the controller may include a user-actuable control for permitting the user to select the vibration frequencies and/or amplitudes for optimally suspending in the gas the type of powder currently being used in the inhaler. The user-actuable control is pre-calibrated with the controller to cause the controller to adjust the frequency and/or amplitude of actuating electricity supplied to the vibrator to be that necessary for vibrating the type of powder selected by the user-actuable control in such a way as to optimally suspend at least a portion of the powder into the gas. The user-actuable control may include selection gradations in terms of the average size of the powder particles to be suspended in the gas, and/or in terms of desired vibration frequencies and amplitudes. Typically, for commonly used powdered medications of 0.5 to 10 micron size, more typically 1 to 5 micron size, vibration frequency would be adjusted to at least about 12 KHz, in order to optimally suspend such commonly used powdered medications in the gas. Of course, vibration frequency and amplitude may be adjusted to optimize suspension of the powdered medication being used.

Advantageously, the piezoelectric vibrator in this embodiment of the invention does not include the many moving mechanical parts of prior art electromechanical vibrator in the inhalers such as disclosed in Wilke et al. Thus, the vibrator in this embodiment does not require the frequent maintenance required by vibrator devices such as disclosed in Wilke et al. Further advantageously, the controller and user-actuable control of this embodiment of the present invention permit the vibration amplitude and frequency imparted to the powder to be quickly and easily adjusted for optimal delivery of different types of powders to the user without the inconvenience of having to disassemble and alter the physical components of this embodiment.

A second embodiment of the invention includes a controllable vibrator for vibrating the powder and a controller for controlling the vibrator. The controller controls the vibrator based, at least partially, upon at least one detected characteristic of the user's inhalation gas stream in and through the inhaler. The detected characteristics of the gas stream upon which the controller bases its control of the vibrator may include the detected velocity, flowrate, and/or pressure of the inhalation gas stream. The vibrator in this second embodiment may comprise a piezoelectric vibrator. Additionally, the controller of this second embodiment may control the vibrator by automatically actuating the vibrator when the at least one detected characteristic of the gas stream has a magnitude that exceeds a minimum threshold value therefor indicative of inhalation by the user, and by automatically deactivating the vibrator when the magnitude of the at least one detected characteristic is less then the minimum threshold.

This second embodiment may also include a plurality of gas inlets and at least one one-way valve in at least one of the inlets. The valve is adapted to permit flow of gas into the inhaler therethrough upon inhalation of gas from the inhaler.

This second embodiment may also include a dispenser for dispensing the powder for being vibrated. The dispenser dispenses the powder based upon control signals supplied to the dispenser by the controller. The controller generates these control signals based upon, at least in part, the at least one detected characteristic of the gas stream. The dispenser may dispense the powder directly to the surface of the vibrator.

Advantageously, these features of this second embodiment permit this embodiment to be able to commence dispensing and vibration of the powder simultaneously with inhalation by the user. Additionally, the one-way valve of this second embodiment prevents outflow of powder from the inhaler unless except during inhalation by the user. These features permit the powdered medication to be delivered to the user with much less waste and with a greater degree of dosage control than is possible according to the prior art.

In a third embodiment of the invention, a vibrator is provided for vibrating the powder. A controller is provided for controlling supply (i.e., frequency and/or amplitude) of actuating electricity to the vibrator based, at least in part, upon detected power transfer characteristics of the vibrator. Preferably, in this third embodiment, the detected power transfer characteristics upon which the controller bases its control of the supply of power include whether the maximum power is being transferred to the vibrator. The controller automatically adjusts the supply of power to the vibrator so as to maximize the detected power transferred to the vibrator.

Advantageously, it has been found that the powder is optimally suspended in the gas when detected power transferred to the vibrator is maximized. Thus, by utilizing the aforesaid automatic feedback and control features of this third embodiment of the present invention, the powder may be optimally suspended in the gas with little to no user interaction with the inhaler (i.e., the user does not need to adjust the frequency and/or amplitude of vibration himself).

Another embodiment of the inhaler of the present invention includes a piezoelectric vibrator for vibrating the drug, which may be a pure micronized drug or a micronized drug on a carrier, in a container such as a blister pack so as to deaggregate clumps of the drug or separate the micronized drug from the carrier particles. Since the intent of the invention is to ensure consistency in the bioavailability of the drug from dose-to-dose, the amount of energy coupled into the powder is sufficient to cause deaggregation to happen and maintain the powder in a fluidized state, but is not so high as to prematurely eject clumps of powder into the air stream that is flowing across the blister.

In yet another embodiment, a vibrator having a vibrating diaphragm is provided for imparting vibrations to the powder. The vibrator may include electrostatic, electromagnetic, or magnetostrictive means for rapidly deflecting the diaphragm. The magnetostrictive means uses at least one ferromagnetic member adapted to change its dimensions and/or shape in the presence and as a function of the magnetic flux applied to the member by a magnetic flux generating means. The magnetic flux generating means may comprise a permanent magnet and a coil, or an electromagnetic means for generating and applying the magnetic flux to the ferromagnetic member.

The micronized particle sizes of interest are then lifted out of the fluidized powder by the passing air stream. In a preferred embodiment of the invention, an electrostatic field that is established across the air stream. By controlling the strength of the electrostatic field only particle sizes of interest are introduced into the air stream. The larger size particles are left behind in the container. This reduces the inconsistency associated with the bioavailability of the drug because of the large particles being deposited into the mouth or throat as is common with devices described in prior art.

Reference is made to the drawings, wherein like numerals depict like parts, and wherein:
Figure 1 is a perspective view of one embodiment of the inhaler of the present invention.
Figure 2 is a rear plane view of the inhaler shown in Figure 1.
Figure 3 is a longitudinal cross-sectional schematic view of the preferred embodiment of Figure 1.
Figure 4 is a functional block diagram of the vibration control system of the embodiment of Figure 1.
Figure 5 is a perspective view of a second preferred embodiment of the inhaler of the present invention.
Figure 6 is a functional block diagram of the vibration control system of the embodiment of Figure 5.
Figure 7A and 7B are side elevations of another embodiment of an inhaler of the present invention;
Figure 8 is an end view of the embodiment of Figure 7;
Figures 9A and 9B is an enlarged cross-sectional views of the embodiment of Figure 7;
Figure 10 is a functional schematic diagram of the electrical/electronic system used in the inhaler of Figure 7;
Figure 11 is a schematic representation of a preferred vibrator driver circuit used for exciting the piezoelectric vibrator;
Figures 12A and 12B are schematic representations of the electrostatic voltage generation circuit used in the inhaler of Figure 7; and
Figure 13 is a view similar to Figure 9A, and showing yet another embodiment of the present invention.

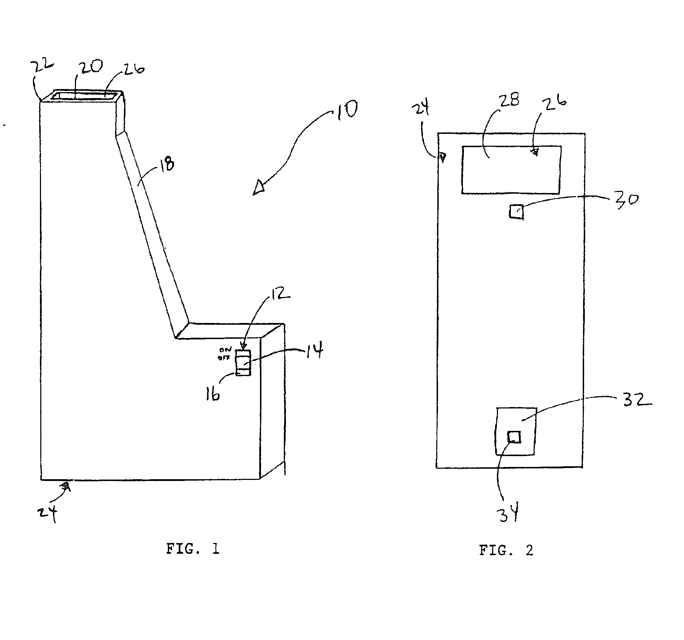

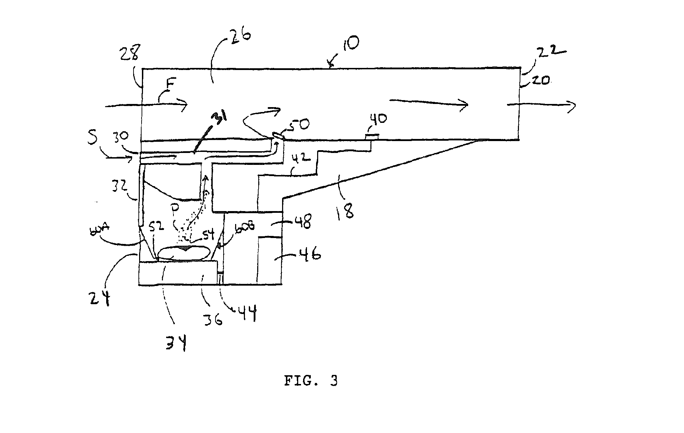

Referring to Figures 1-4, one preferred embodiment 10 of the inhaler of the present invention will now be made. Inhaler 10 includes a hard plastic or metal housing 18 having a generally L-shaped longitudinal cross-section. Housing 18 includes four air flow openings 20, 28, 30, and 32 (whose function in this embodiment of inhaler 10 of the present invention will be described more fully below). Inhaler 10 includes a main air flow passage 26 which extends the length of the housing 18 from the front 22 (at opening 20) to the rear 24 thereof (at opening 28) and has a generally square-shaped transverse cross-section, so as to permit air flow therethrough (denoted by arrow F in Figure 1).

Secondary air conduit 31 is generally L-shaped and runs longitudinally from opening 30 in the rear 24 surface of the housing 18 to main passage 26. One-way flow valve 50 is mounted to the inner surface 33 of the main passage 26 via a conventional spring-biased hinge mechanism (not shown), which is adapted to cause the valve 50 to completely block air flow S through the conduit 31 to the main passage 26 when the pressure of the air flow F in the main passage 26 is below a predetermined threshold indicative of inhalation through the passage 26 by a user.

Powder dispensing chamber 51 is formed in housing 18 for holding a capsule 34 of powder medication to be inhaled. Housing 18 includes a moveable panel portiorr 32 in the rear 24 for permitting the capsule 34 to be introduced into the chamber 51 and placed on the seating 52 of vibration means 36 between guiding means 60A, 60B. Preferably, means 36 comprises a hard plastic or metallic protective shell 37 enclosing a piezoelectric vibrator 90. Preferably, vibrator 90 is mechanically coupled through the shell 37 via a disk (not shown) to the drug cartridge 34 so as to permit maximum vibratory energy to be transmitted from the vibrator 90 through the shell 37 to the cartridge 34. Guiding means 60A, 60B includes two surfaces which slant downwardly toward the seating 52 so as to permit easy introduction and retention of the capsule on the seating 52 in the chamber 51. Removable panel 32 includes another air inlet 34 for permitting additional air flow S2 from the chamber 51 through conduit 61 into conduit 31 during inhalation by the user. Preferably, panel 32 and housing 18 include conventional mating mounting means (not shown) for permitting the panel 32 to be removably resecurable to the housing by the user between introduction of fresh (i.e., completely full) capsules and removal of spent (i.e., empty) capsules.

Inhaler 10 also includes a conventional miniature air stream velocity or pressure sensor 40 mounted on the inner surface of the conduit 26 so as to sense the speed and/or pressure of the air stream F. Preferably, sensor 40 comprises a conventional spring-loaded flapper-yield switch which generates electronic signals indicative of the speed and/or pressure of the air stream F in the conduit 26, and transmits those signals via electrical connection 42 to electronic control circuitry 48 contained in housing 18 for controlling actuation of the vibrator means based upon those signals.

Preferably, the control circuitry 48 is embodied as an application specific integrated circuit chip and/or some other type of very highly integrated circuit chip. As will be described more fully below, the control circuitry 48 determines the amplitude and frequency of actuating power to be supplied from conventional power source 46 (e.g., one or more D.C. batteries) to the piezoelectric vibrator to thereby control vibration of the vibrator. The actuating power is supplied to the piezoelectric element 90 via electrical connection 44 between the vibrator and the circuitry 48.

Piezoelectric element 90 is made of a material that has a high-frequency, and preferably, ultrasonic resonant vibratory frequency (e.g., about 15 to 50 kHz), and is caused to vibrate with a particular frequency and amplitude depending upon the frequency and/or amplitude of excitation electricity applied to the peizoelectric element 90. Examples of materials that can be used to comprise the piezoelectric element 90 include quartz and polycrystalline ceramic materials (e.g., barium titanate and lead zirconate titanate). Advantageously, by vibrating the piezoelectric element 90 at ultrasonic frequencies, the noise associated with vibrating the piezoelectric element 90 at lower (i.e., non-ultrasonic) frequencies can be avoided.

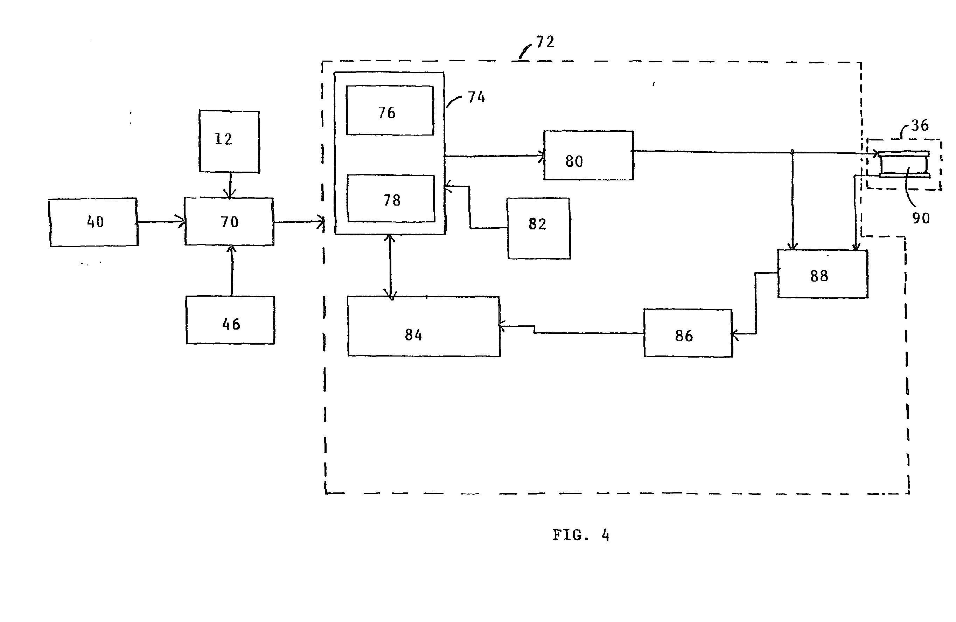

Turning to Figure 4, the various functional components and operation of the control circuitry 48 will now be described. As will be understood by those skilled in the art, although the functional components shown in Figure 4 are directed to an analog realization of the control circuitry 48, the components of Figure 4 could be appropriately modified to realize control circuitry 48 in a digital embodiment without departing from this embodiment 10 of the present invention.

Control circuitry 48 preferably includes actuation controller 70 and vibratory feedback control system 72. Acutation controller 70 comprises a,conventional switching mechanism for permitting actuating power to be supplied from the power source 46 to the control system 72 depending upon the signals supplied to it from sensor 40 and the state of the power switch 12. In other words, controller 70 permits actuating power to be supplied from the source 46 to the system 72 when the sliding indicator bar 14 of switch 12 is set to the "ON" position in channel track 16 and the inhalation sensor 40 supplies signals to the controller 70 that indicate that the inhalation is occuring through the main passage 26. However, controller 70 does not permit actuating power to flow from the source to the system 72 when either the switch 12 is set to "OFF" or the signals supplied to the controller 70 from the sensor 40 indicate that inhalation is not taking place through the conduit 26.

When controller 70 first permits actuating power to be supplied from the source 46 to the feedback control system 72, the system 72 enters an initialization state wherein controllable means for supplying a predetermined frequency and amplitude of actuating electricity 74 is caused to generate control signals for causing conventional pump circuit 80 to generate an initial desired frequency and amplitude of actuating electricity based upon stored values thereof stored in the initialization memory means 82. Preferably, means 74 comprises conventional frequency sweep generator and frequency generator means 76 and 78, respectively. The signals generated by means 74 are then supplied to charge pump circuit 80 to cause circuit 80 to supply the piezoelectric element 90 with actuating electricity specified by the signals.

Preferably, the initial frequency and amplitude of actuating electricity supplied to the piezoelectric element 90 is pre-calibrated to cause the piezoelectric element 90 to vibrate at its resonance frequency when no powder cartridge or powder is placed on the means 36. As will be appreciated by those skilled in the art, maximum transfer of vibratory power from the piezoelectric element to the powder in the container 34 takes place when the piezoelectric element vibrates at its resonant frequency. It has been found that this results in maximum de-aggregation and suspension of the powder from the container 34 into the air to be inhaled by the user. However, when the container 36 or powder is placed on the vibrator means 36, the weight and volume of the powder container, and the weight, volume, and particular size of the powder to be suspended by the piezoelectric element can change the vibration characteristics of the piezoelectric element, and cause the piezoelectric element to vibrate at other than its resonant frequency. This can result in reduced vibratory energy transfer to the powder from the piezoelectric element, and thereby, lessen the efficiency of the piezoelectric element in de-aggregating and suspending the powder in the air inhaled by the user.

The feedback control system 72 of the present invention overcomes this problem. In control system 72, after the initial frequency and amplitude of actuating electricity are supplied to the piezoelectric element 90, the frequency generating means 74 systematically generates control signals indicative of many different amplitudes and frequencies of electricity for being supplied to the piezoeletric element 90 by the circuit 80. As the generating means 74 "cycles through" the different frequencies and amplitudes, the instantanous power transfer characteristics of the piezoelectric element 90 for each of these different frequencies and amplitudes are determined by the detector 88, which transmits this information to the peak power detector 86. Peak detector 86 analyzes the instantaneous power transfer characteristics of the piezoelectric element 90 and signals the sample and hold feedback controller 84 when the power transfer characteristics are at local maxima. The controller 84 correlates these local maxima with the frequencies and amplitudes commanded by the generator 74 to be supplied to the piezoelectric element 90.

After the frequency generator 74 has finished its sweep through the frequencies and amplitudes of power supplied to the piezoelectric element 90, the controller 84 causes the generator 74 to cycle through the frequencies and amplitudes of power that resulted in local maxima, and then determines which of these frequencies and amplitudes results in optimal power transfer characteristics through the piezoelectric element 90.

In operation of embodiment 10, the drug-containing package 34 is punctured and inserted onto the surface 52 of vibrator 36 in chamber 51 in the manner described previously. The power switch is placed in the "ON" position and the user inhales air through the conduit 26, air flow F is generated through conduit 26. This causes one-way valve 50 to deflect to admit air flow S through opening 30 into conduit 26, and also causes air flow S2 through opening 34 and chamber 51 into conduit 26. The inhalation of air stream F is sensed by sensor 40 and is signaled to actuation controller 70, which causes power to be supplied to the controller 72. The controller 72 then adjusts the amplitude and frequency of actuating power supplied to the piezoelectric element until they are optimized for the best possible de-aggregation and suspension of the powder P from the capsule into the air stream F via air flows S and S2.

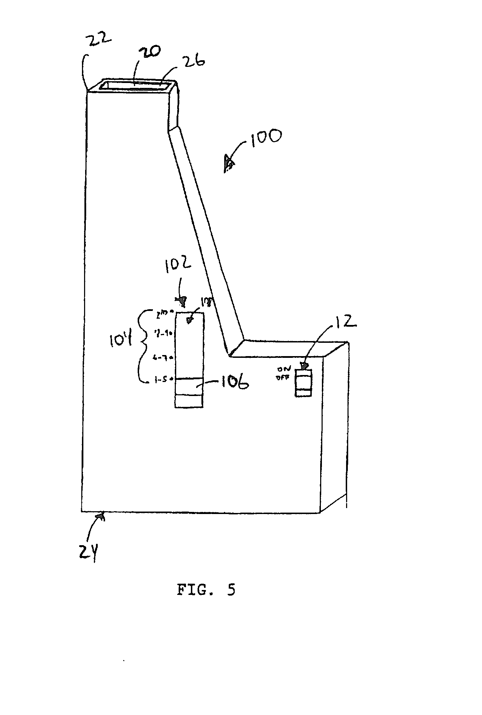

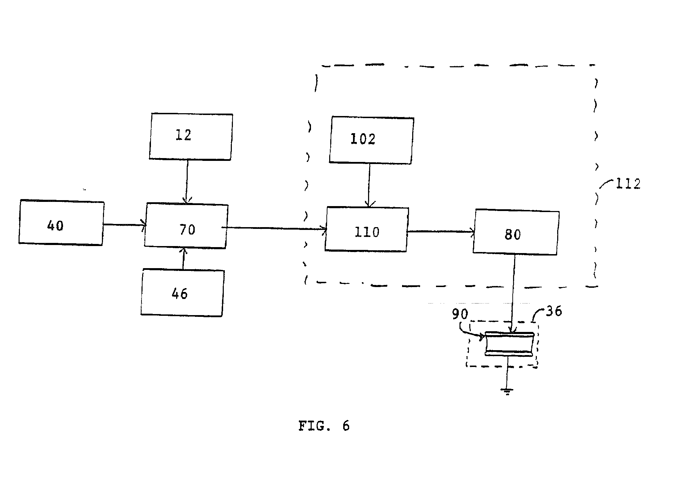

Turning to Figures 5-6, a second preferred embodiment 100 of the present invention will now be described. It should be appreciated that unless specifically indicated to the contrary, the components and operation of embodiment 100 are substantially identical to those of embodiment 10. In embodiment 10, the feedback controller 72 of embodiment 10 is replaced with a pre-calibrated controller 112. Controller 112 includes precalibrated frequency/amplitude control signal generator 110 which supplies control signals to pump circuit 80 based upon signals supplied to it from user-actuable frequency/amplitude controller switch 102. Switch 102 includes indicator means 106 slidably mounted in channel track 108 and permits a user to command the generator 110 to generate control signals for causing the pump 80 to supply amplitude and frequencies of actuating power to the piezoelectric element for optimally suspending powders of differing, pre-calibrated particular sizes. For example, when the selector switch is set to the "1-5" position, the generator 110 may be pre-programmed to generate control signals for causing the pump 80 to supply to the piezoelectric element 90 actuating electricity having a frequency and amplitude for optimally suspending powder particles between about 1 and 5 microns in size. Of course, it will appreciated that the pre-calibrated optimal frequency and amplitude values programmed in generator 110 are based upon an expected weight and volume the container 34 and powder contained therein. Thus, in order for the inhaler 100 to be able to optimally suspend the powder, the weight and volume of the container 34 and powder contained therein cannot different significantly from the expected values thereof upon which the pre-calibration values programmed into the generator 110 were based.

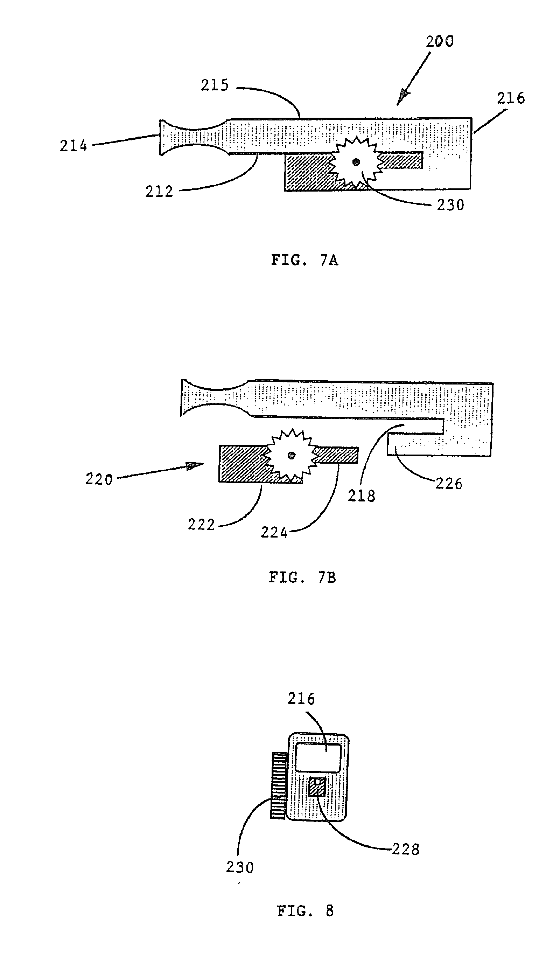

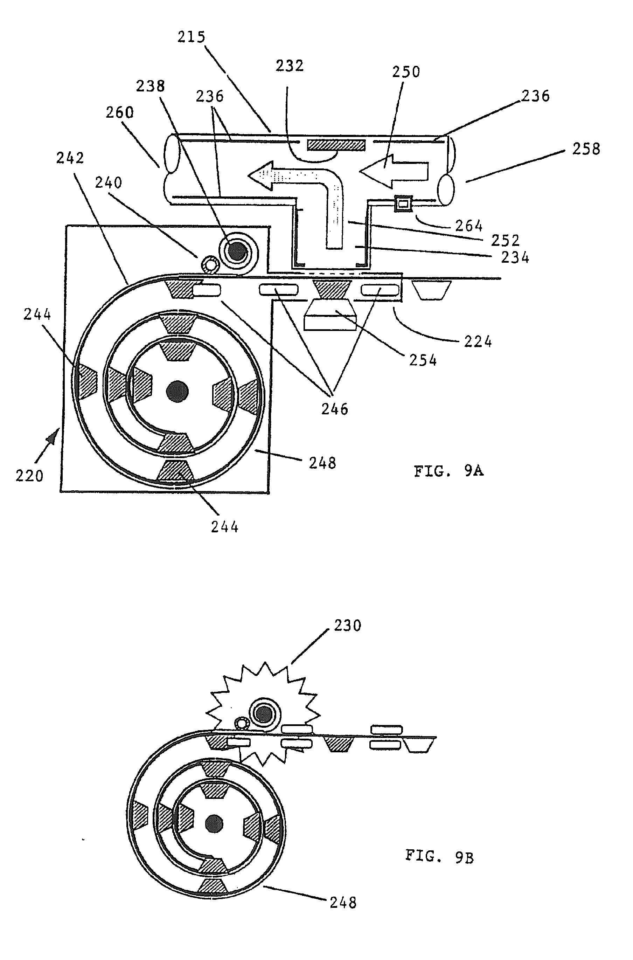

Referring to Figures 7-12, another and preferred embodiment 200 of the inhaler of the present invention will now be described. The inhaler 200 comprises a housing 212 having a mouthpiece 214 at one end of leg 215 thereof, and an air inlet opening 216 at the opposite end of the same leg 215. The leg 215 forms a hollow channel that is unobstructed between the opening 214 and 216. Referring in particular to Figure 9A, a passageway 234 is formed in leg 215 intermediate of mouthpiece 214 and inlet 216. This passageway communicates with a drug cartridge 220. Opposite this passageway 234 is a high frequency piezoelectric 254 vibrator located in member 226 of the inhaler housing 212. An inhalation sensor 264 is also located between the passageway 234 and the inlet 216 of the inhaler.

Disposable cartridge 220 comprises an outer housing 222 which includes a tab 224 for slidably mounting in recess 218 formed integrally to the housing 212. Drug cartridge 220 includes a coiled tape 248 carrying a plurality of spaced blisters or wells 244 for carrying a dry powder medicament. A release film 242 covers and seals wells 244. Tape 248 is formed as a coil, and threaded between guide platen 246 and pinch roller 240. Pinch roller 240 is driven by a take-up spool 238 which in turn is driven by the thumbwheel 230. In use, as the thumbwheel 230 is turned, it peels the release film 242 from the tape 248 to expose the wells 244 carrying the drug, and the release film is collected by the take-up spool 238. This collection of the release film advances a new well carrying the drug over the piezoelectric vibrator 254 housed in location 226 of the inhaler housing 215. Tape 248 also preferably includes detent means or the like for indexing the tape so a selected well 244 is automatically positioned over the piezoelectric element 254.

Referring to Figure 9A, a passageway 234 allows the selected well 244 to communicate with the hollow channel 215. Passageway 234 should be of sufficient length to serve the purpose of avoiding to introduce the drug prematurely into the air stream 250, which could be zero, if the wells 244 have sufficient depth. Above the exposed well 244, in the passageway 234, is an electrostatic plate 232 on the inside wall of the hollow channel 215. Channel 215 also includes an inhalation sensor 264, the purpose of which is to detect the flow of an air stream from the inlet 216 to the mouthpiece 214. This sensor signal is used to sequence the electronic control of the inhaler to ensure repeatable performance.

A brief description of the sequence of operation is as follows. A new well 244 carrying the drug is advanced forward and positioned over the piezoelectric element 254. A power switch 272 on the inhaler housing 212 (not shown in figure) is turned on. This connects the power source 274 to the electronics. At this point the output of the actuation controller circuit 270 is not enabled. When a minimum air-stream 250 flowrate is established, the actuation controller 270 enables the vibrator driver circuit 276 and the electrostatic voltage generator circuit 278. The high frequency vibrations set up by the piezoelectric vibrator 254 are coupled through the well 244 into the powder. These high frequency vibrations deaggregate the powder in the well and keep the powder in a fluidized state. The electrostatic plate 232 sets up an electrostatic field across the fluidized powder. The deaggregated particles, which carry an electrostatic charge, experience an electrostatic force and are lifted up by this field set up by the electrostatic plate 232. This electrostatic force experienced by the charged particles is counteracted by the mass of the particles. Smaller particles which are unable to counteract their mass are lifted up toward the electrostatic plate 232, while larger size particles which cannot counteract their mass are left behind in the well 244. The voltage applied to the electrostatic plate 232 is selected so that only deaggregated drug particles, i.e. smaller size particles of choice, are lifted towards the electrostatic plate 232, where enter the air stream 250 and are carried to the mouthpiece 214. On the other hand, the electrostatic force on the charged particles is not so strong as to enable the particles to make it across the air stream 250 to the electrostatic plate 232.

The inner surface of the channel 215 and the passageway 234 is metalized and connected to ground. Without this metalization 236, static charges could build up on the inside surfaces of the channel 215 and passageway 234, and attract charged drug particles as they are lifted out of the well 244 and make their way to the mouthpiece 214, thus reducing reproducibility of delivery from dose-to-dose pf the inhaler.

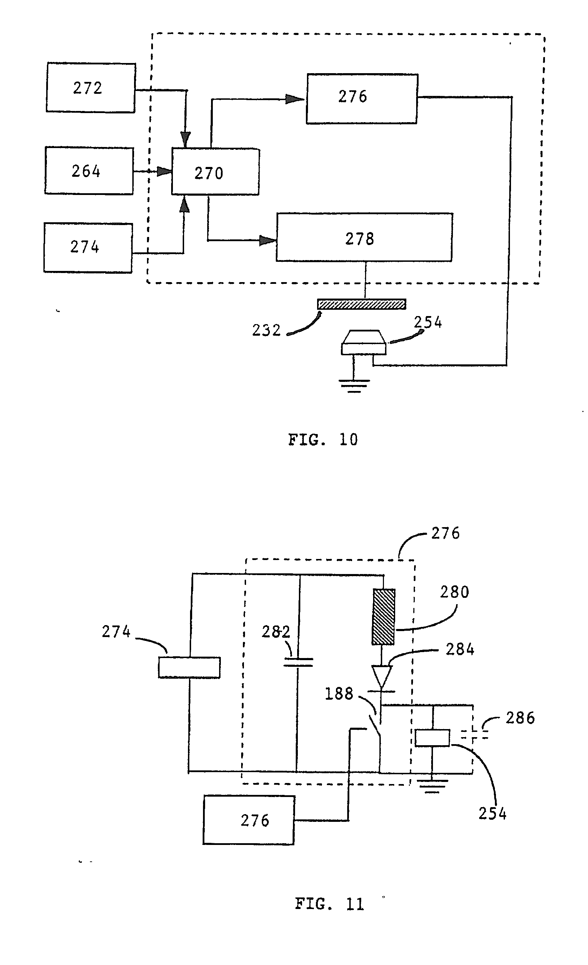

Figure 11 shows a preferred schematic embodiment of the vibrator drive circuit 276. This configuration helps couple energy efficiency to the high frequency piezoelectric vibrator 254, while at the same time generating the necessary high voltages for driving the piezo. This circuit configuration enables the inhaler to be designed as a portable unit and enables it to achieve very small package dimensions relative to similar units described in the prior art. The circuit consists of an inductance 280 in series with a diode 284 and switch 288. The switch 288 may be of a bipolar transistor or a field effect transistor or any other means capable of switching at high frequency in an efficient manner, i.e. without loss of considerable energy. Elements 280, 284 and 288 are connected across a bypass capacitor 282 capable of carrying high frequency currents. The piezoelectric element 254 is connected across switch 288. The actuation controller 270 switches the switch 288 on and off at the appropriate frequency necessary to drive the piezoelectric vibrator. The sequence of operation is as follows: When switch 288 is turned ON, current builds up in the inductance 280. When switch 288 is turned OFF, the inductance 280 then resonates with the internal capacitor of the piezo element 254. This series resonant circuit efficiently couples the energy stored in the inductance to the piezo element while at the same time generating the high voltage necessary to drive it. If necessary, the internal capacitance of the piezo may be supplemented by an external capacitor 286 to control the energy/voltage transfer characteristics to the piezo 254.

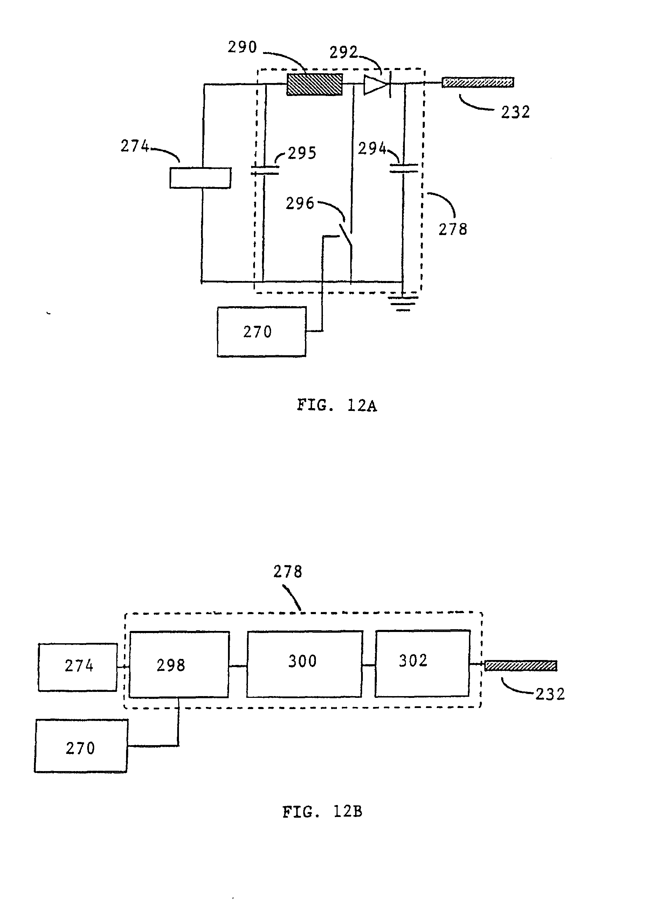

Figures 12A and 12B show the schematic representation of the electrostatic voltage generation circuit 278. The Figure 12A schematic is suitable for generating low voltages, for example, less than 200 volts. The actuation controller 270 turns a switch 296 ON and OFF at a frequency which will result in efficient build up of voltage across capacitor 294. When switch 296 turns ON, current builds up in an inductance coil 290. When switch 296 is turned OFF, the inductance pumps this energy into the output capacitor 294 by generating a voltage one diode drop higher than the output voltage. The inductor 290 continues to maintain this voltage till all the energy stored in it is transferred to capacitor 294. The electrostatic plate 232 is connected to capacitor 294. The voltage generated across capacitor 294 sets up the electrostatic field across the fluidized and deaggregated powder in the well 244. Capacitor 295 is a bypass capacitor for carrying the high frequency switching currents.

If a higher electrostatic field is required, it may be necessary to use the schematic shown in Figure 12B. Here a high frequency inverter 298 is coupled to a high frequency transformer 300. The output of transformer 300 feeds into voltage multiplier circuit 302 for stepping up the voltage to the required level. The output of the voltage multiplier circuit is connected to the electrostatic plate.

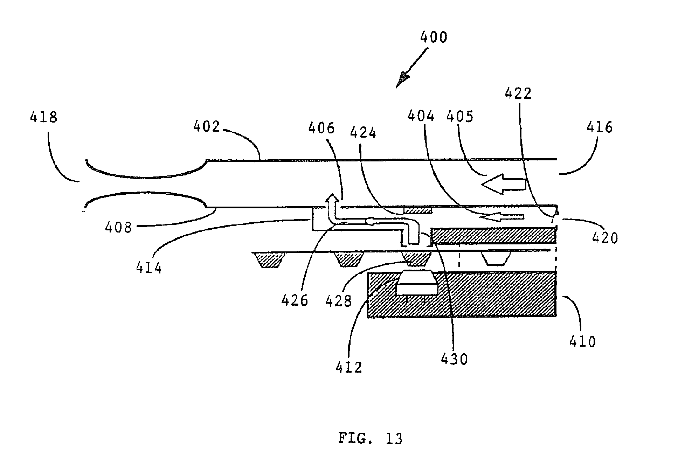

Figure 13 shows yet another embodiment 400 of the present invention. This embodiment would be suitable for those applications where we wish to further reduce the possibility of air turbulence picking up the partially deaggregated powder from the blister wells. This embodiment consists of a housing 402 which includes a mouthpiece 418 at one end of a main leg 408, and a main air inlet 416 at the opposite end of the leg 408. An opening 406 is provided at one end of a secondary leg 414 that opens into the main leg 408, and an air inlet 420 is provided at the opposite end of the secondary leg 414. Housing 402 also includes a high frequency vibrator, such as piezoelectric vibrator 412, located in member 410 of the housing 402. A secondary channel 430 communicates with the secondary leg 414 at one end and a drug container 428 at the other end. Channel 430 allows the selected drug container to communicate with the secondary leg 414. Directly under the drug container is the vibrator 412.

The Figure 13 embodiment operates as follows: A new well 428 carrying the drug is advanced forward and positioned over the piezoelectric vibrator 412. A power switch on the inhaler housing (not shown in the figure) is turned on. This connects the power to the electronics. When the patient inhales a main air flow 405 and a secondary air flow 404 is established. The secondary air flow 404 causes a flapper valve 422 to open and enables the vibrator drive circuit 276 and the electrostatic generator circuit 278 (see Figure 10). The flapper valve functions as an air flow sensor. The high frequency vibrator 412 deaggregates the powder in the container 428 and keeps it in a fluidized state. An electrostatic plate 424 sets up an electrostatic field across the fluidized powder. The deaggregated particles which carry a charge are lifted up towards electrostatic plate 424. The resulting particle stream 426 is introduced into the air stream 404 and is carried forward and introduced into the main air stream 405.

It will be appreciated that although the foregoing detailed description proceeded with reference being made to the preferred embodiments and methods of use, the present invention is not intended to be limited to these preferred embodiments and methods of use. Rather, the present invention is of broad scope and is intended to be limited only as set forth in the accompanying claims.

## Claims

1. A dry powder inhaler comprising a housing (18, 402) having means (36, 412) for deaggregating a dry powder, and a primary air flow passageway (26, 416) in which the deaggregated powder is picked up and carried for introduction, via a mouthpiece (22, 418), into a patient, wherein the means for deaggregating (36, 412) is in communication with a chamber (51, 430) in which the powder is deaggregated, and the primary air flow passageway (26, 416) communicates with said chamber (51, 430) so that deaggregated powder is picked up and carried for introduction into a patient, **characterized in that** (i) the means for deaggregating coniprises a piezoelectric vibrator (36, 412), and (ii) said inhaler further comprises a secondary air flow passageway (31, 414) of reduced cross - sectional size connecting the chamber (51, 430) and the primary air flow passageway (26, 416).

2. An inhaler according to claim 1, **characterized by** further comprising at least one detector (40) for detecting velocity and/or pressure of air flow (F) in said passageway (26).

3. An inhaler according to claim 2, **characterized by** further comprising a controller (70) adapted to automatically actuate or deactivate said vibrator (36) in response to signals from the detector (40).

4. An inhaler according to claim 3, **characterized in that** said controller (70) includes an actuable control for controlling actuation of said vibrator (36) based upon a selected type of powder.

5. An inhaler according to any of claims 1-4, **characterized in that** at least a portion of the inner surface of the inhaler housing (215) is metallized (236).

6. An inhaler according to any of claims 1-5, **characterized by** further comprising a high frequency inverter (298), a high frequency transformer (300) and a voltage multiplier circuit (302) for generating a voltage for driving said piezoelectric vibrator (36, 412).

7. An inhaler according to claim 1, **characterized by** further comprising an inhalation sensor (264) for activating and deactivating said piezoelectric vibrator (36, 412), in response to inhalation of air (250) by the user.

8. An inhaler according to any of claims 1-7, **characterized by** further comprising said medication in powder form is supplied to said inhaler in a sealed container pnor to use.

9. An inhaler according to any of claims 1-8, **characterized by** further comprising electrostatic potential means (232) for separating the deaggregated powder by size and driving size-separated deaggregated powder of interest into the air flow passageway.

10. An inhaler according to any of claims 1-9, further **characterized by** a secondary airflow passage by which air is sucked from outside the housing at an inlet (S).
